(19) Europäisches Patentamt
European Patent Office
Office européen des brevets

(11) Publication number: 0 086 916
A1

# (12) EUROPEAN PATENT APPLICATION

(21) Application number: 82306355.7

(22) Date of filing: 30.11.82

(51) Int. Cl.[3]: C 07 D 249/08
C 07 D 233/60, C 07 D 405/06
//A01N43/64, A01N43/50

(30) Priority: 29.01.82 GB 8202598

(43) Date of publication of application: 31.08.83 Bulletin 83/35

(84) Designated Contracting States: AT BE CH DE FR GB IT LI NL SE

(71) Applicant: IMPERIAL CHEMICAL INDUSTRIES PLC
Imperial Chemical House Millbank
London SW1P 3JF(GB)

(72) Inventor: Crowley, Patrick Jelf
56 Ellis Road
Crowthorne Berkshire(GB)

(72) Inventor: Williams, John Cyril
36 Wylam, Great
Holland Bracknell Berkshire(GB)

(74) Representative: Alner, Henry Giveen Hamilton et al,
Imperial Chemical Industries PLC Legal Department: Patents Thames House North Millbank
London SWIP 4QG(GB)

## (54) Novel process for preparing heterocyclic compounds useful as pesticides.

(57) A novel process for preparing fungicidally active compounds of formula:

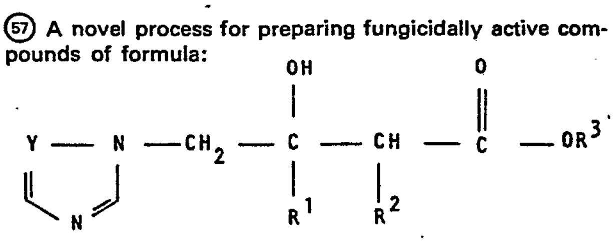

wherein Y is −CH= or =N−, $R^1$ is alkyl, cycloalkyl or optionally substituted phenyl and $R^2$ and $R^3$, which may be the same or different, are hydrogen, alkyl, cycloalkyl, phenyl or benzyl or together form a lactone ring, comprises reacting a phenacyl triazole with an ester enolate anion.

EP 0 086 916 A1

## NOVEL PROCESS FOR PREPARING HETEROCYCLIC COMPOUNDS USEFUL AS PESTICIDES

This invention relates to a process for preparing triazole compounds useful as fungicides. using them.

The present invention provides a process for preparing triazole compounds having the general formula (I):

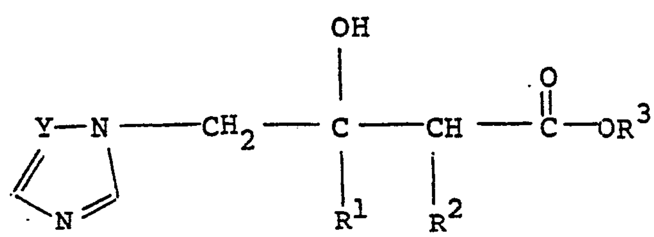

wherein Y is -CH= or =N-, $R^1$ is alkyl, cycloalkyl or optionally substituted phenyl and $R^2$ and $R^3$, which may be the same or different, are hydrogen, alkyl, cycloalkyl, (eg. cyclopropyl, cyclopentyl or cyclohexyl), optionally substituted phenyl or optionally substituted benzyl, or together form a lactone ring; which comprises bringing into reaction a compound of formula (II) with the enolate anion (IV) generated by treatment of (III) with a strong base such as lithium diisopropylamide.

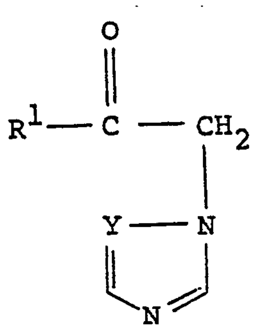

(II)

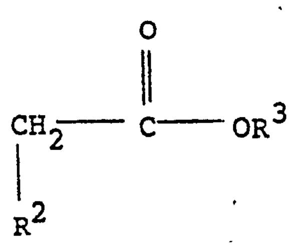

(III)

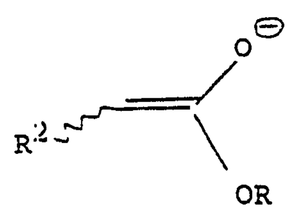

(IV)

The compounds of general formula (II) may be made by methods set out in the literature.

Suitably the enolate anions of general formula (IV) are generated by low temperature treatment of compounds (III) with lithium diisopropylamide; which itself can be produced *in situ* by the reaction of *n*-butyl lithium with diisopropylamine.

The advantages of this process are the very much higher yields, and better reproducability than the process described in UK Patent Application No 8027451. (European Application 81303271.1). The process is also potentially applicable to a wide variety of different types of ester.

The salts and metal complexes of the compounds of general formula (I) can be prepared from the latter in known manner. For example, the complexes can be made by reacting the uncomplexed compound with a metal salt in a suitable solvent.

The compounds can contain chiral centres so that the compounds produced by the foregoing process are generally obtained in the form of mixtures. However, these and other mixtures can be separated into the individual isomers by methods set out in the art.

The alkyl groups in the foregoing formulae can be a straight or branched groups having 1 to 6, eg. 1 to 4, carbon atoms; such groups are methyl, ethyl, propyl (*n*- or *iso*-propyl) and butyl (*n*-, *sec*-, *iso*- or *t*-butyl).

Examples of suitable substituents for an optionally substituted phenyl group (ie. for $R^1$,$R^2$ and/or $R^3$ and for the phenyl moiety of a benzyl group (ie. for $R^2$ and/or $R^3$) are halogen, alkyl, alkoxy, nitro and phenyl. The alkyl moiety (ie. the α-carbon atom) of the benzyl can be substituted with for example one alkyl (eg. methyl or ethyl). Suitably the phenyl and benzyl are unsubstituted or substituted with 1, 2 or 3 ring substituents as defined above. Examples of these groups are phenyl, benzyl, α-methylbenzyl, 2-, 3- or 4-chloro-phenyl, 2,4- or 2,6-dichlorophenyl, 2-, 3- or 4-fluoro-phenyl, 2-, 3- or 4-bromophenyl, 2-, 3- or 4-methoxyphenyl, 2,4-dimethoxyphenyl, 2-, 3- or 4-ethoxy-phenyl, 2-, 3- or

4-nitrophenyl, 2-, 3- or 4- methylphenyl, 2-, 3- or 4-trifluoromethylphenyl, 2-chloro-4-methylphenyl, 2-chloro-4-methoxyphenyl, 2-chloro-4-cyano-phenyl, and 4-phenyl-phenyl (4-biphenylyl), and the corresponding ring substituted benzyl and α-methylbenzyl groups.

In a further aspect, therefore, the invention provides a process, as hereinbefore defined, for preparing a compound of the general formula (I) above, or a stereoisomer thereof, wherein $R^1$ is a straight or branched chain alkyl group having from 1 to 6 carbon atoms or is phenyl optionally substituted with halogen, alkyl, alkoxy, nitro, phenyl or phenoxy; $R^2$ and $R^3$ are together an alkylene bridging group, or each represents hydrogen, straight or branched chain alkyl groups having from 1 to 6 carbon atoms, phenyl or benzyl each optionally substituted with halogen, alkyl, alkoxy, nitro, phenyl or phenoxy, the alkyl (ie. the α-carbon atom) of the benzyl being optionally substituted with alkyl; and Y is =CH- or =N-.

In a preferred aspect the invention provides a process, as hereinbefore defined, for preparing a compound of formula (I) above, or a stereoisomer thereof, wherein $R^1$ is an alkyl group containing from 1 to 4 carbon atoms or is halophenyl, $R^2$ is hydrogen or methyl; $R^3$ is an alkyl group containing from 1 to 4 carbon atoms and Y is =N- or =CH-.

The salts can be salts with inorganic or organic acids eg. hydrochloric, nitric, sulphuric, acetic, 4-toluenesulphonic or oxalic acid. The esters are suitably alkanoates (eg. acetates) with the $R^3$ group suitably alkyl (eg. methyl or ethyl), aryl (eg. phenyl) or aralkyl (eg. benzyl).

Suitably the metal complex is one including, as the metal, copper, zinc, manganese or iron. It preferably has the general formula:

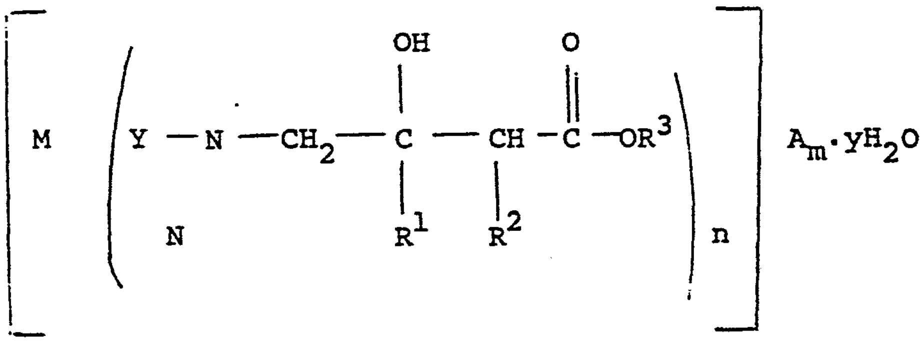

wherein Y, $R^1$,$R^2$ and $R^3$ are as defined above, M is a metal, A is an anion (eg. a chloride, bromide, iodide, nitrate, sulphate or phosphate anion), n is 2 or 4, y is 0 or an integer of 1 to 12, and m is an integer consistent with valency.

Examples of the compounds which may be produced by the invention process are shown in Table 1. These conform to formula I.

TABLE 1

| COMPOUND NO | $R^1$ | $R^2$ | $R^3$ | Y | MELTING POINT (°C) |
|---|---|---|---|---|---|
| 1 | 4-chlorophenyl | H | ethyl | =N- | 84 |
| 2 | 4-chlorophenyl | H | tertiary butyl | =N- | 94-6 |
| 3 | 4-fluorophenyl | H | tertiary butyl | =N- | 92-4 |
| 4 | 4-chlorophenyl | $*-CH_2-CH_2-$ | | =N- | 163-4 |
| 5 | tertiary butyl | H | tertiary butyl | =N- | 140 |
| 6 | 2,4-dichloro-phenyl | H | tertiary butyl | =N- | 138-9 |

TABLE 1 CONTINUED

| COMPOUND NO | $R^1$ | $R^2$ | $R^3$ | Y | MELTING POINT (°C) |
|---|---|---|---|---|---|
| 7 | 2,4-dichloro-phenyl | H | normal butyl | =N- | |
| 8 | 2,4-dichloro-phenyl | H | tertiary butyl | =CH- | |
| 9 | 2,4-dichloro-phenyl | $CH_3$ | tertiary butyl | =N- | isomer A oil<br>isomer B 110-112 |
| 10 | 2,4-dichloro-phenyl | $CH_3$ | tertiary butyl | =CH- | |
| 11 | 4-chlorophenyl | $CH_3$ | tertiary butyl | =N- | |
| 12 | 4-chlorophenyl | $CH_3$ | tertiary butyl | =CH- | |

TABLE 1 CONTINUED

| COMPOUND NO | $R^1$ | $R^2$ | $R^3$ | Y | MELTING POINT (°C) |
|---|---|---|---|---|---|
| 13 | 4-(4-chloro-phenyl)phenyl | H | tertiary butyl | =N- | |
| 14 | 4-(4-chloro-phenyl)phenyl | H | tertiary butyl | =CH- | |

* ie. $R^2$ and $R^3$ together form a bridging group

The foregoing compounds, salts and metal complexes are active fungicides, particularly against the diseases:-

Piricularia oryzae on rice

Puccinia recondita, Puccinia striiformis and other rusts on wheat, Puccinia hordei, Puccinia striiformis and other rusts on barley, and rusts on other hosts eg. coffee, apples, vegetables and ornamental plants

Plasmopara viticola on vines

Erysiphe graminis (powdery mildew) on barley and wheat and other powdery mildews on various hosts such as Sphaerotheca fuliginea on cucurbits (eg. cucumber), Podosphaera leucotricha on apples and Uncinula necator on vines

Helminthosporium spp. and Rhynchosporium spp. on cereals

Cercospora arachidicola on peanuts and other Cercospora species on for example sugar beet, bananas and soya beans

Botrytis cinerea (grey mould) on tomatoes, strawberries, vines and other hosts

Phytophthora infestans (late blight) on tomatoes

Venturia inaequalis (scab) on apples

Some of the compounds have also shown a broad range of activities against fungi in vitro. They have activity against various post-harvest diseases on fruit (eg. Penicillium digatatum and italicum on oranges and Gloeosporium musarum on bananas). Further some of the compounds are active as seed dressings against: Fusarium spp., Septoria spp., Tilletia spp. (ie. bunt, a seed borne disease of wheat), Ustilago spp., Helminthosporium spp. on cereals, Rhizoctonia solani on cotton and Corticium sasakii on rice. The compounds can move acropetally in the plant tissue. Moreover, the compounds can be volatile enough

to be active in the vapour phase against fungi on the plant.

The compounds are also useful for the treatment of candidiasis and human dermatophyte infections.

The compounds may be used as such for fungicidal purposes but are more conveniently formulated into compositions for such usage.

EXAMPLE 1

This Example illustrates the preparation of the compound having the structure

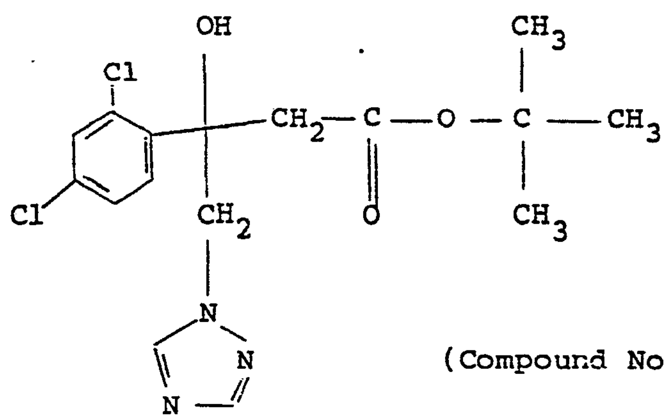

(Compound No 6 of Table I)

n-Butyl lithium (6.5 ml of 1.6 m solution in hexane) was slowly added dropwise to diisopropylamine (1.05g) stirred in dry THF (60 ml) at -20°C under argon. After stirring for 0.5 hr, t-butylacetate (1.16g) in dry THF (10 ml) was slowly added dropwise to the solution cooled to -40°C, and after completion of the addition the reaction mixture was stirred at -40°C for 0.5 hr, 2,4-dichlorophenacyltriazole (2.23g) in dry THF (30 ml) was slowly added dropwise over 10 min, and the mixture stirred at -40° for 0.5 hr. The temperature was allowed to rise to 5° over 0.5 hr. The temperature was allowed to rise to 5° over

2 hr. The reaction was carefully quenched with water and extracted with ether, and the ethereal extract dried over magnesium sulphate, and evaporated to yield an oil which crystallised on scratching in petrol (1.30 g, mpt 138-9°).

NMR and IR were identical to the previously made sample.

EXAMPLE 2

This Example illustrates the preparation of two isomers of the compound having the structure

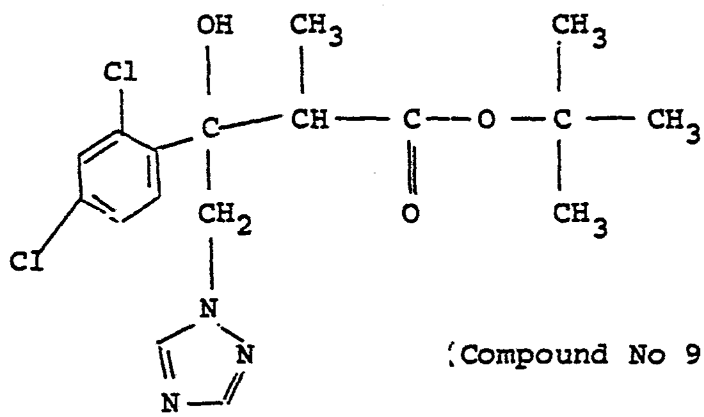

(Compound No 9 of Table I)

n-Butyl lithium (7 ml of a 1.6m solution in hexane) was slowly added dropwise to diisopropylamine (1.0g) stirred in dry THF (60 ml) at -20°C under argon. After stirring for 0.5 hr, t-butylpropionate (1.30g) in dry THF (10 ml) was slowly added dropwise to the solution cooled to -40°, and after completion of the addition the reaction mixture was stirred at -40° for 0.5 hr, 2,4- Dichloro-phenacyltriazole (1.8g) in dry THF (30 ml) was slowly added dropwise over 10-15 min. and the mixture stirred at -40° for 0.5 hr, and then warmed to 5° over 2-5 hr. The reaction was carefully quenched with water, extracted with ether and the ethereal extract dried over magnesium sulphate. Evaporation of the solvent yielded an oil which

was chromatographed, on silica gel eluting with ethyl acetate. Isomer A was eluted first (RF approx 0.7) and was an oil (0.40g). Isomer B was eluted second (RF approx 0.45) and was a solid (0.35g, mpt 110-112°).

Isomer A   R 156623

NMR ($CDCl_3$) $\delta$ : 1.0(d,3H), 1.66(S,9H), 4.0(q,1H), 4.92 (d,1H), 5.75(d,1H), 5.95(S,1H), 7.66(dd,1H), 7.90(d,1H), 8.16(d,1H), 8.28(S,1H), 8.62(S,1H)

IR (liquid form) 3400 (bs), 1725 and 1695 $cm^{-1}$

Isomer B   R 156624

NMR ($CDCl_3$) $\delta$ : 1.20 (s,9H), 1.48(d,3H), 4.16(q,1H), 4.80 (d,1H) 5.32(d,1H), 5.55(S,1H), 7.50(dd,1H), 7.72-7.92 (H,2H), 8.14(s,1H), 8.50(S,1H)

IR (mijol)   3480 (sharp), 1705 $cm^{-1}$

HGHA/jlw
10.11.82
SPEC299

1. A process for preparing compounds having the general formula (I):

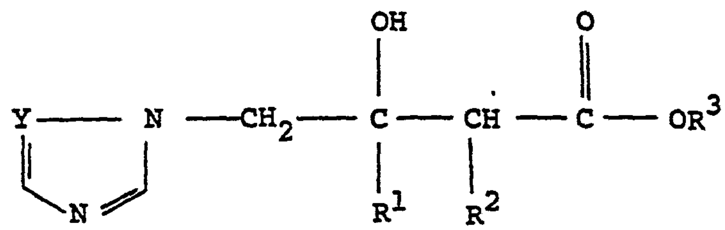

(I)

wherein Y is -CH= or =N-, $R^1$ is alkyl, cycloalkyl or optionally substituted phenyl and $R^2$ and $R^3$, which may be the same or different, are hydrogen, alkyl, cycloalkyl, (eg. cyclopropyl, cyclopentyl or cyclohexyl), optionally substituted phenyl or optionally substituted benzyl, or together form a lactone ring which comprises bringing into reaction a compound of general formula (II) with the enolate anion of formula (IV) generated by treatment of (III) with a strong base such as lithium diisopropylamide.

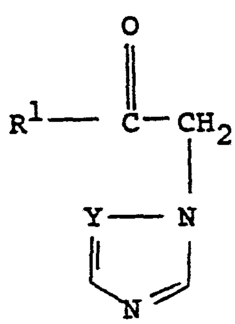

(II)

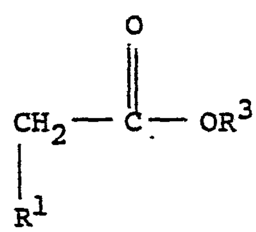

(III)

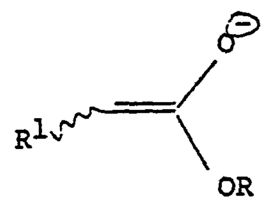

(IV)

2. A process as claimed in claim 1 wherein the enolate anions of formula (IV) are prepared by the low temperature treatment of compounds of formula (III) with lithium diisopropylamide.

3. A process as claimed in claim 1 or claim 2 wherein, in formula (I), $R^1$ is a straight or branched chain alkyl group having from 1 to 6 carbon atoms or is phenyl optionally substituted with halogen, alkyl, alkoxy, nitro, phenyl or phenoxy; $R^2$ and $R^3$ are together an alkylene bridging group, or each represents hydrogen, straight or branched chain alkyl groups having from 1 to 6 carbon atoms, phenyl or benzyl each optionally substituted with halogen, alkyl, alkoxy, nitro, phenyl or phenoxy, the alkyl (ie. the $\alpha$-carbon atom) of the benzyl being optionally substituted with alkyl; and Y is =CH- or =N-.

4. A process as claimed in any of the preceding claims wherein, in formula (I), $R^1$ is an alkyl group containing from 1 to 4 carbon atoms or is halophenyl, $R^2$ is hydrogen or methyl; $R^3$ is an alkyl group containing from 1 to 4 carbon atoms and Y is =N- or =CH-.

5. A process as claimed in any of the preceding claims wherein when any of $R^1$, $R^2$ and $R^3$ in formula (I) is an alkyl group it is a straight or branched chain group having 1 to 6 carbon atoms and especially 1 to 4 carbon atoms such as methyl, ethyl, propyl (_n_- or _iso_-propyl) and butyl (_n_- _sec_-, _iso_- or _t_-butyl).

6. A process as claimed in any of the preceding claims wherein, in formula (I), when any of $R^1$, $R^2$ and $R^3$ is a phenyl group and/or when any of $R^2$ and $R^3$ is a benzyl group, they are phenyl, benzyl, $\alpha$-methylbenzyl, 2-, 3- or 4-chlorophenyl, 2,4- or 2,6-dichlorophenyl, 2-, 3- or 4-fluorophenyl, 2-, 3- or 4-bromophenyl, 2-, 3- or 4-methoxyphenyl, 2-4-dimethoxyphenyl, 2-, 3- or 4-ethoxyphenyl, 2-, 3- or 4-nitrophenyl, 2-, 3- or 4-methylphenyl, 2-, 3- or 4-trifluoromethylphenyl, 2-chloro-4-methylphenyl, 2-chloro-4-methoxyphenyl, 2-chloro-4-cyanophenyl, and 4-phenyl-phenyl (4-biphenylyl), or a correspondingly ring substituted benzyl or α-methylbenzyl group.

7. A process as claimed in any of the preceding claims comprising the further step of converting the compound so produced into a salt or metal complex as hereinbefore described.

8. Compounds as defined in any of the preceding claims whenever produced by a process as defined in any of the preceding claims.

9. The processes of Examples 1 and 2 as described hereinbefore.

10. A process as claimed in any of the preceding claims and further comprising the step of separating any racemic mixture so produced into the individual isomers.

European Patent Office

**0086916**
Application number

# EUROPEAN SEARCH REPORT

EP 82 30 6355

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,Y | EP-A-0 046 633 (I.C.I.) * Claims * | 1-10 | C 07 D 249/08<br>C 07 D 233/60<br>C 07 D 405/06 //<br>A 01 N 43/64<br>A 01 N 43/50 |
| Y | HOUBEN-WEYL: "Methoden der organischen Chemie", 4th edition. vol. VI/la, section 2, Alkohole II, 1980, Georg Thieme Verlag, New York, USA<br>H. KROPF: "VII. Herstellung von Alkoholen durch Aufbaureaktionen", pages 928-152<br>* Pages 1322-1324 * | 1-10 | |

TECHNICAL FIELDS SEARCHED (Int. Cl. 3)

C 07 D 249/00
C 07 D 233/00

The present search report has been drawn up for all claims

| Place of search | Date of completion of the search | Examiner |
|---|---|---|
| THE HAGUE | 17-05-1983 | CREMERS K. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82